(19) Europäisches Patentamt
European Patent Office
Office européen des brevets

(11) **EP 4 345 775 B1**

(12) **EUROPÄISCHE PATENTSCHRIFT**

(45) Veröffentlichungstag und Bekanntmachung des Hinweises auf die Patenterteilung:
**06.08.2025   Patentblatt 2025/32**

(21) Anmeldenummer: **22199236.5**

(22) Anmeldetag: **30.09.2022**

(51) Internationale Patentklassifikation (IPC):
***G06V 20/69*** *(2022.01)*     ***G06V 10/82*** *(2022.01)*

(52) Gemeinsame Patentklassifikation (CPC):
**G06V 20/695; G06V 10/82; G06V 20/698**

(54) **VERFAHREN ZUR DETEKTION WENIGSTENS EINES FLUORESZENZMUSTERS AUF EINEM IMMUNFLUORESZENZBILD EINES BIOLOGISCHEN ZELLSUBSTRATS**

METHOD FOR DETECTING AT LEAST ONE FLUORESCENCE PATTERN ON AN IMMUNOFLUORESCENCE IMAGE OF A BIOLOGICAL CELL SUBSTRATE

PROCÉDÉ DE DÉTECTION D'AU MOINS UN MOTIF FLUORESCENT SUR UNE IMAGE IMMUNOFLUORESCENTE D'UN SUBSTRAT DE CELLULE BIOLOGIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**03.04.2024   Patentblatt 2024/14**

(73) Patentinhaber: **EUROIMMUN Medizinische Labordiagnostika AG**
**23560 Lübeck (DE)**

(72) Erfinder:
• **HOCKE, Jens**
  **23560 Lübeck (DE)**
• **KRAUTH, Jens**
  **23560 Lübeck (DE)**
• **GERLACH, Stefan**
  **23560 Lübeck (DE)**
• **KRAUSE, Christopher**
  **23560 Lübeck (DE)**
• **HAHN, Melanie**
  **23560 Lübeck (DE)**
• **VOIGT, Jörn**
  **23560 Lübeck (DE)**
• **SCHMIDT, Enno**
  **23538 Lübeck (DE)**
• **VAN BEEK, Nina**
  **23538 Lübeck (DE)**

(56) Entgegenhaltungen:
**EP-A1- 4 016 082     US-A1- 2019 371 425**

• **JIANG GUAN-TING ET AL: "Automatic HEp-2 cell segmentation in indirect immunofluorescence images using deep learning", SPIE SMART STRUCTURES AND MATERIALS + NONDESTRUCTIVE EVALUATION AND HEALTH MONITORING, 2005, SAN DIEGO, CALIFORNIA, UNITED STATES, SPIE, US, vol. 11792, 20 April 2021 (2021-04-20), pages 1179205 - 1179205, XP060140805, ISSN: 0277-786X, ISBN: 978-1-5106-4548-6, DOI: 10.1117/12.2590426**

**Beschreibung**

Die Erfindung betrifft ein Verfahren zur digitalen Bildverarbeitung geeignet zur Detektion wenigstens eines Fluoreszenzmusters auf einem Immunfluoreszenzbild eines biologischen Zellsubstrats.

Zu dem Zwecke einer Informationsgewinnung für eine diagnostische Fragestellung können biologische Substrate, wie beispielsweise ein Zellsubstrat, mittels einer sogenannten Immunfluoreszenz prozessiert und dann die sich ergebenden Immunfluoreszenzbilder des Substrates analysiert werden.

Eine indirekte Immunfluoreszenzmikroskopie ist ein in-vitro-Test zum Nachweis zirkulierender humaner Antikörper gegen bestimmte Antigene, um eine diagnostische Fragestellung beantworten bzw. einschätzen zu können. Derartige Antigene sind beispielsweise in bestimmten Bereichen von Zellsubstraten, insbesondere Hautschichten von Primaten, vorhanden. Als Substrat dient also ein Zellsubstrat, welches mit einer Patientenprobe in Form von Blut oder verdünntem Blut oder aber Blutserum oder verdünntem Blutserum inkubiert wird. Die Patientenprobe weist also potenziell bestimmte Antikörper auf, welche Ausdruck eines Vorhandenseins einer Erkrankung des Patienten sein können. Solche primären bzw. spezifischen Antikörper können dann Antigene des Substrats binden. Derartig gebundene primäre Antikörper können dann dadurch markiert werden, dass in einem weiteren Inkubationsschritt sogenannte sekundäre Antikörper, vorzugsweise Anti-Human-Antikörper, an die gebundenen primären Antikörper anbinden und später dadurch sichtbar gemacht werden können, dass die sekundären Antikörper mit einem Fluoreszenzfarbstoff markiert sind. Ein solcher Fluoreszenzfarbstoff ist vorzugsweise ein grüner Fluoreszenzfarbstoff, insbesondere der Fluoreszenzfarbstoff FITC. Eine solche Bindung eines primären Antikörpers gemeinsam mit einem fluoreszenzmarkierten sekundären Antikörper kann dann später dadurch sichtbar gemacht werden, dass das Substrat mit Anregungslicht einer bestimmten Wellenlänge bestrahlt wird und somit die gebundenen Fluoreszenzfarbstoffe zur Emission von Fluoreszenzstrahlung angeregt werden.

Ein biologisches Zellsubstrat kann hierbei insbesondere ein Zellausstrich von Zelllinien, eine Kolonie von Zelllinien und/oder biologisches Gewebe mit biologischen Gewebezellen, insbesondere aus einem Gewebeschnitt, sein.

Abhängig vom Fluoreszenzmuster auf dem jeweilgen Substrat können die detektierten Autoantikörper Krankheitsgruppen zugeordnet werden. Es ergibt sich also die Aufgabe, mittels digitaler Bildverarbeitung im Zuge einer Immunfluoreszenzmikroskopie bei einem in der vorgeschriebenen Weise inkubierten Zellsubstrat mittels digitaler Bildverarbeitung einen oder mehrere Fluoreszenzmustertypen in einem Fluoreszenzbild einer indirekten Immunfluoreszenzmikroskopie zu detektieren.

Insbesondere bei einer Betrachtung von Erkrankungen wie bullöse Autoimmundermatosen, welche die menschliche Haut betreffen, kann mittels Immunfluoreszenzbildanalyse eine Informationsgewinnung dahingehend geleistet werden, ob an sich überhaupt irgendeine bullöse Autoimmundermatose vorliegt und vorzugsweise auch eine Differenzierung, welche Art oder Untergruppe von bullösen Autoimmundermatosen vorliegt.

Hierzu werden als biologisches Zellsubstrat mitunter Ösophagus-Abschnitte eines Affen und/oder sogenannte Salt-split Skin Substrate einer Immunfluoreszenz unterzogen.

Eine sogenannte Salt-split Skin ist ein biologisches Zellsubstrat in Form eines Hautsubstrates eines Primaten, vorzugsweise eines Affen. Bei einem solchen Hautsubstrat wird eine Separation der Epidermis von der Dermis durch Injektion von einer Salzlösung herbeigeführt, insbesondere 1molare Natriumchloridlösung.

Das Dokument US2019/0371425A1 offenbart ein Klassifikationssystem und ein Klassifikationsverfahren im Zusammenhang mit einer Detektion von Autoantikörpern mittels Immunfluoreszenz. Offenbart wird hierin eine Eingabeeinheit, in welche mehrere Zellimmunfluoreszenzbilder eingegeben werden und wobei ein Prozessor eine Mehrheit von convolution neuronal networks anwendet, um eine Klassifikation der Bilder durchzuführen.

Das Dokument EP4016082A1 offenbart ein Verfahren und eine Vorrichtung zum Detektieren einer Präsenz eines Fluoreszenzmustertyps auf einem Organschnitt mittels Immunfluoreszenzmikroskopie, wobei eine Teilfläche des Fluoreszenzbildes bestimmt wird, welche für eine Ausbildung des Fluoreszenzmustertyps, welcher detektiert werden soll, relevant ist.

Das Dokument D3 (Jiang Guan-Ting et al: "Automatic HEp-2 cell segmentation in indirect immunofluorescence images using deep learning", Spie smart structures and materials + nondestructive evaluation and health monitoring, 2005, San Diego, California, United States, Spie, US, Bd. 11792, 20. April 2021 (2021-04-20), Seiten 1179205-1179205, XP060140805, ISSN: 0277-786X, DOI: 10.1117/12.2590426, ISBN: 978-1-5106-4548-6) offenbart eine automatische Segmentierung von indirekten Immunfluoreszenzbilder mittels Deep Learning, insbesondere Convolutional Neuronal Networks, wobei die Bilder sogenannte HEp-2-Zellen repräsentieren.

Aufgabe der vorliegenden Erfindung ist es, ein Immunfluoreszenzbild eines biologischen Zellsubstrats in einer automatisierten Weise unter Verwendung wenigstens eines neuronalen Netzes dahingehend zu analysieren, ob wenigstens ein Fluoreszenzmuster in dem Immunfluoreszenzbild des Zellsubstrats präsent ist.

Die erfindungsgemäße Aufgabe wird gelöst durch das erfindungsgemäße Verfahren. Erfindungsgemäß erfolgt zunächst ein Bereitstellen eines Immunfluoreszenzbilder, welches eine Färbung eines biologischen Zellsubstrats durch einen Fluoreszenzfarbstoff repräsentiert.

Ferner erfolgt erfindungsgemäß ein Bestimmen einer Segmentierungsinformation, welche wenigstens einen ersten und einen zweiten Segmentierungsbereich aufweist. Die jeweiligen Segmentierungsbereiche repräsentieren jeweils einen jeweiligen Zellsubstratbereich des Zellsubstrates. Diese Segmentierungsinformation wird mittels Segmentieren des Immunfluoreszenzbildes unter Verwendung eines ersten neuronalen Netzes bestimmt.

Ferner folgt ein Bestimmen eines Grenzbereiches, welcher einen Übergang von dem ersten Zellsubstratbereich hin zu dem zweiten Zellsubstratbereich in dem Fluoreszenzbild repräsentiert, auf Basis der Segmentierungsinformationen. Ferner erfolgt ein Selektieren mehrerer Teilbilder des Immunfluoreszenzbildes entlang des bestimmten Grenzbereichs. Ferner erfolgt ein Bestimmen eines Konfidenzmaßes einer Präsenz des Fluoreszenzmusters auf Basis der mehreren Teilbilder mittels eines zweiten neuronalen Netzes.

Zur Darlegung möglicher Vorteile des erfindungsgemäßen Verfahrens wird nun genauer auf verschiedene Aspekte dieses erfindungsgemäßen Verfahrens eingegangen.

Die grundlegende Fragestellung, ein Immunfluoreszenzbild hinsichtlich einer Präsenz eines zu erwartenden Fluoreszenzmusters zu analysieren, ist in der Immunfluoreszenz hinreichend bekannt. Da es je nach diagnostischer Fragestellung unterschiedliche zu erwartende Fluoreszenzmuster gibt, ist ein Bildverarbeitungsverfahren für ein Immunfluoreszenzbild eben dann besonders leistungsfähig, wenn es auf die zu erwartenden Fluoreszenzmuster für eine Analyse unter Verwendung eines neuronalen Netzes besonders angepasst ist. Eine einfache Prozessierung eines großen, gesamten Immunfluoreszenzbildes mittels eines neuronalen Netzes, welches sämtliche Bildinformationen des gesamten Immunfluoreszenzbildes berücksichtigt, erfordert ein hochkomplexes neuronales Netz, welches besonders viele Rechenoperationen durchführen muss. Ferner ist ein solches neuronales Netz mit einer hohen Komplexität und vielen Freiheitsgraden besonders schwer zu trainieren, da eine hohe Menge an Trainingsdaten benötigt wird, um eine Generalisierung des neuronalen Netzes zu erreichen.

Daher weicht das vorgeschlagene erfindungsgemäße Verfahren explizit von einem solchen Ansatz einer Gesamtanalyse eines gesamten Immunfluoreszenzbildes mittels eines neuronalen Netzes ab, um besonders leistungsfähig zu sein.

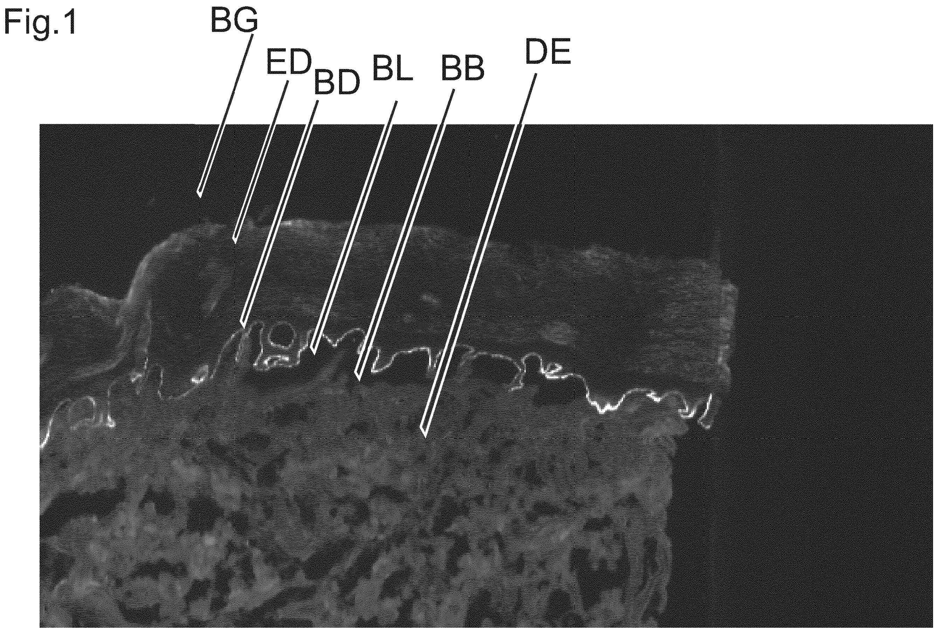

Ein beispielhaftes Immunfluoreszenzbild FB eines Zellsubstrates, hier vorzugsweise in Form einer Salt-split Skin, ist in der Figur 1 dargestellt.

Das Immunfluoreszenzbild FB zeigt für das Zellsubstrat einen Zellsubstratbereich ED, welcher eine Epidermis präsentiert. Ferner zeigt das Immunfluoreszenzbild FB einen Bereich DE, welcher eine Dermis repräsentiert. Bei der Salt-split Skin ist ein sogenannter Zwischenbereich bzw. eine sogenannte Blase klar erkennbar als Substratbereich BL. Der erste Zellsubstratbereich ist also vorzugsweise die Epidermis, wobei der zweite Zellsubstratbereich vorzugsweise der Zwischenraum bzw. die Blase zwischen Dermis und Epidermis ist.

Die Figur 1 zeigt ferner in dem Fluoreszenzbild FB einen sogenannten Hintergrundbereich oder Background BG.

Ein Übergang bzw. Grenzbereich zwischen der Epidermis ED und der Blase BL wird vorzugsweise auch als Blasendach BD bezeichnet. In dem Immunfluoreszenzbild der Figur 1 liegt eine Fluoreszenz entlang dieses Blasendaches BD als ein Grenzbereich vor.

Ferner ergibt sich ein Grenzbereich BB als der sogenannte Blasenboden, welches also ein Grenzbereich zwischen der Blase BL (bzw. dem Zwischenraum) und der Dermis DE ist. In dem Beispiel des Fluoreszenzbildes FB der Figur 1 liegt keine Fluoreszenz entlang des Blasenbodens vor.

Liegt eine Fluoreszenz entlang wenigstens eines der Grenzbereiche vor, also entlang nur des Blasendachs, nur des Blasenbodens oder aber des Blasendachs und des Blasenbodens, so ist dies ein Hinweis auf eine prinzipielle Pemphigoiderkrankung. Wird also erfindungsgemäß in einem Grenzbereich eine Präsenz eines Fluoreszenzmusters detektiert, so ist dies ein Hinweis auf eine prinzipielle Pemphigoiderkrankung.

Wird vorzugsweise in einem bestimmten Grenzbereich, welches vorzugsweise das Blasendach BD ist, eine Präsenz eines Fluoreszenzmusters detektiert, so kann für die Fragestellung möglicher infrage kommender Erkrankungen eine Information dahingehend gewonnen werden, dass bestimmte Gruppen von Erkrankungen ausgeschlossen werden können. Daher ist eine Information, ob entlang des Grenzbereiches bzw. des Blasendachs BD eine Fluoreszenz vorliegt, für einen Kliniker von Vorteil. Es kann also dann, wenn der zuvor bestimmte Grenzbereich zwischen bestimmten Zellsubstratbereichen, wie beispielsweise der Epidermis ED und dem Zwischenraum bzw. der Blase BL, analysiert wird und eine Präsenz eines Fluoreszenzmusters detektiert wird, möglicherweise eine bestimmte Untergruppe von bullösen Autoimmundermatosen in Betracht gezogen werden, wie z.B. das bullöse Pemhigoid, welche in Europa die häufigste bullöse Autoimmundermatose ist. Mit anderen Worten: Ist eine Fluoreszenz entlang des sogenannten Blasendachs BD präsent, so kann dies ein Hinweis auf zwei bestimmte Zielantigene sein, welches wiederum ein Hinweis auf ein bullöses Pemphigoid sein kann.

Wird vorzugsweise in einem bestimmten Grenzbereich, welches vorzugsweise der Blasenboden BB ist, eine Präsenz eines Fluoreszenzmusters detektiert, so kann dies ein Hinweis auf seltenere Pemphigoiderkrankungen außerhalb der Gruppe des bullösen Pemphigoids sein, wie z.B. auf eine Epidermolysis bullosa acquisita.

Vorzugsweise erfolgt ferner ein Ausgeben des Konfidenzmaßes.

Vorzugsweise erfolgt das Bestimmen des Konfidenzmaßes in der Weise, dass zunächst für ein jeweiliges Teilbild ein jeweiliges Teilbildkonfidenzmaß mittels des zweiten neuronalen Netzes bestimmt wird und das dann das Konfidenzmaß auf Basis der Teilbildkonfidenzmaße bestimmt wird.

Vorzugsweise werden die mehreren Teilbildbereiche zufallsbasiert aus dem Immunfloreszenzbild entlang des Grenzbereiches selektiert.

Vorzugsweise wird durch das zweite neuronale Netz ferner für ein jeweiliges Teilbild ein jeweiliger Helligkeitswert bestimmt und dann ein Gesamthelligkeitswert auf Basis dieser jeweiligen Helligkeitswerte bestimmt.

Vorzugsweise erfolgt ferner ein Bestimmen der Segmentierungsinformation in der Weise, dass die Segmentierungsinformation den ersten, den zweiten sowie ferner einen dritten Segmentierungsbereich aufweist. Der dritte Segmentierungsbereich repräsentiert vorzugsweise einen dritten Zellsubstratbereich. Auch hier folgt das Bestimmen der Segmentierungsinformation mittels Segmentieren des Immunfloreszenzbildes unter Verwendung des ersten neuronalen Netzes. Vorzugsweise sind die mehreren Teilbilder (TB1, ..., TBX) Teilbilder einer ersten Art. Ferner erfolgt vorzugsweise ein Bestimmen eines zweiten Grenzbereiches, welcher einen zweiten Übergang von dem zweiten Zellsubstrat hin zu dem dritten Zellsubstrat repräsentiert, auf Basis der Segmentierungsinformation. Vorzugsweise erfolgt ferner ein Selektieren mehrerer Teilbilder zweiter Art aus dem Immunfloreszenzbild entlang des zweiten Grenzbereiches. Vorzugsweise erfolgt ein Bestimmen eines zweiten Konfidenzmaßes einer Präsenz eines zweiten Floreszenzmusters auf Basis der mehreren Teilbilder zweiter Art mittels eines dritten neuronalen Netzes.

Vorgeschlagen wird ferner ein Computerprogrammprodukt umfassend Befehle, die bei der Ausführung des Programms durch einen Computer diesen veranlassen, das Verfahren zur digitalen Bildverarbeitung durchzuführen.

Vorgeschlagen wird ferner ein Datenträgersignal, welches das Computerprogrammprodukt überträgt.

Vorgeschlagen wird ferner eine Vorrichtung zur Detektion wenigstens eines Floreszenzmusters auf ein Immunfloreszenzbild eines biologischen Zellsubstrats, aufweisend.

Die Vorrichtung weist ferner wenigstens eine Recheneinheit auf, welche ausgebildet ist, folgende Schritte auszuführen: Bestimmen einer Segmentierungsinformation aufweisend wenigstens einen ersten und einen zweiten Segmentierungsbereich, wobei die Segmentierungsbereiche jeweils einen jeweiligen Zellsubstratbereich repräsentieren, mittels Segmentieren des Immunfloreszenzbildes unter Verwendung eines ersten neuronalen Netzes, Bestimmen eines Grenzbereiches, welcher einen Übergang von dem ersten Zellsubstratbereich hin zu dem zweiten Zellsubstratbereich in dem Floreszenzbild repräsentiert, auf Basis der Segmentierungsinformation, Selektieren mehrerer Teilbilder aus dem Immunfloreszenzbild entlang des Grenzbereiches, Bestimmen eines Konfidenzmaßes einer Präsenz des Floreszenzmusters auf Basis der mehreren Teilbilder mittels eines zweiten neuronalen Netzes.

Offenbart wird hierin ferner eine Recheneinheit, welche ausgebildet ist im Zuge einer digitalen Bildverarbeitung die Schritte auszuführen: Entgegennehmen eines Immunfloreszenzbildes, welches eine Färbung eines biologischen Zellsubstrats durch einen Floreszenzfarbstoff repräsentiert, Bestimmen einer Segmentierungsinformation aufweisend wenigstens einen ersten und einen zweiten Segmentierungsbereich, wobei die Segmentierungsbereiche jeweils einen jeweiligen Zellsubstratbereich repräsentieren, mittels Segmentieren des Immunfloreszenzbildes unter Verwendung eines ersten neuronalen Netzes, Bestimmen eines Grenzbereiches, welcher einen Übergang von dem ersten Zellsubstratbereich hin zu dem zweiten Zellsubstratbereich in dem Floreszenzbild repräsentiert, auf Basis der Segmentierungsinformation, Selektieren mehrerer Teilbilder aus dem Immunfloreszenzbild entlang des Grenzbereiches, Bestimmen eines Konfidenzmaßes einer Präsenz des Floreszenzmusters auf Basis der mehreren Teilbilder mittels eines zweiten neuronalen Netzes.

Vorgeschlagen wird ferner eine Datennetzwerkvorrichtung, aufweisend wenigstens eine Datenschnittstelle zum Entgegennehmen eines Floreszenzbildes, welches eine Färbung eines Zellsubstrats durch ein Floreszenzfarbstoff repräsentiert sowie ferner aufweisend wenigstens eine Recheneinheit, welche ausgebildet ist im Zuge einer digitalen Bildverarbeitung folgende Schritte auszuführen: Bestimmen einer Segmentierungsinformation aufweisend wenigstens einen ersten und einen zweiten Segmentierungsbereich, wobei die Segmentierungsbereiche jeweils einen jeweiligen Zellsubstratbereich repräsentieren, mittels Segmentieren des Immunfloreszenzbildes unter Verwendung eines ersten neuronalen Netzes, Bestimmen eines Grenzbereiches, welcher einen Übergang von dem ersten Zellsubstratbereich hin zu dem zweiten Zellsubstratbereich in dem Floreszenzbild repräsentiert, auf Basis der Segmentierungsinformation, Selektieren mehrerer Teilbilder aus dem Immunfloreszenzbild entlang des Grenzbereiches, Bestimmen eines Konfidenzmaßes einer Präsenz des Floreszenzmusters auf Basis der mehreren Teilbilder mittels eines zweiten neuronalen Netzes.

Im Folgenden wird die Erfindung anhand spezieller Ausführungsformen ohne Beschränkung des allgemeinen Erfindungsgedankens anhand der Figuren näher erläutert. Dabei zeigen:

Figur 1 ein gesamtes Fluoreszenzbild,

Figur 2a ein weiteres Fluoreszenzbild,

Figure 2b eine Segmentierungsinformation,

Figur 2c Grenzbereiche,

Figur 3 entlang eines Grenzbereiches selektierte Teilbilder eines Fluoreszenzwertes,

Figur 4a zeigt Schritte einer bevorzugten Ausführungsform eines hierin offenbarten Verfahrens,

Figur 4b bevorzugte Teilschritte zur Bestimmung eines Konfidenzmaßes gemäß einer bevorzugten Ausführungsform,

Figur 5 Schritte zur Bestimmung von Teilbildkonfidenzmaßen sowie Teilbildhelligkeitsmaßen als auch eines Konfidenzmaßes und eines Helligkeitsmaßes gemäß einer bevorzugten Ausführungsform der Erfindung,

Figuren 6a, 6b, 6c bevorzugt durchzuführende Schritte eine bevorzugten Ausführungsform des offenbarten Verfahrens,

Figur 7 eine beispielhafte Struktur eines zweiten neuronalen Netzes,

Figur 8 eine bevorzugte Ausführungsform einer erfindungsgemäßen Vorrichtung,

Figur 9 eine bevorzugte Ausführungsformen einer Recheneinheit,

Figur 10 eine bevorzugte Ausführungsform einer erfindungsgemäßen Datennetzvorrichtung,

Figur 11 eine bevorzugte Ausführungsform eines erfindungsgemäßen Computerprogrammproduktes als auch eines vorgeschlagenen Datensignals,

Figur 12 Experimentalergebnisse.

Hierin beschriebene Ausführungsbeispiele sind nicht als Einschränkung der Erfindung zu verstehen. Vielmehr sind im Rahmen der vorliegenden Offenbarung durchaus auch Ergänzungen und Modifikationen möglich, insbesondere solche, die zum Beispiel durch Kombination oder Abwandlung von einzelnen in Verbindung mit den im allgemeinen oder speziellen Beschreibungsteil beschriebenen sowie in den Ansprüchen und/oder der Zeichnung enthaltenen Merkmalen oder Verfahrensschritten für den Fachmann im Hinblick auf die Lösung der Aufgabe entnehmbar sind und durch kombinierbare Merkmale zu einem neuen Gegenstand oder zu neuen Verfahrensschritten bzw. Verfahrensschrittfolgen führen.

Wie zuvor dargelegt zeigt die Figur 1 ein Fluoreszenzbild FB, welches mittels des erfindungsgemäßen Verfahrens analysiert werden kann. Das Fluoreszenzbild FB zeigt eine Fluoreszenz eines Zellsubstrates. Das Zellsubstrat ist vorzugsweise ein Hautsubstrat eines Primaten. Insbesondere weist das Hautsubstrat eine Dermis und eine Epidermis auf. Der Substratbereich ED zeigt den Bereich der Epidermis, wobei der Substratbereich DE den Bereich der Dermis zeigt. Der Zwischenraum zwischen Epidermis und Dermis ist als Zwischenraum bzw. Blase BL zu erkennen. Der Grenzbereich BD als Blasendach zwischen Epidermis und dem Zwischenraum bzw. Blase BL zeigt in diesem Beispiel eine Fluoreszenz auf.

Der Grenzbereich des sogenannten Blasenbodens BD zwischen dem Zwischenraum bzw. Blase BL und der Dermis DE zeigt in diesem Beispiel keine Fluoreszenz auf.

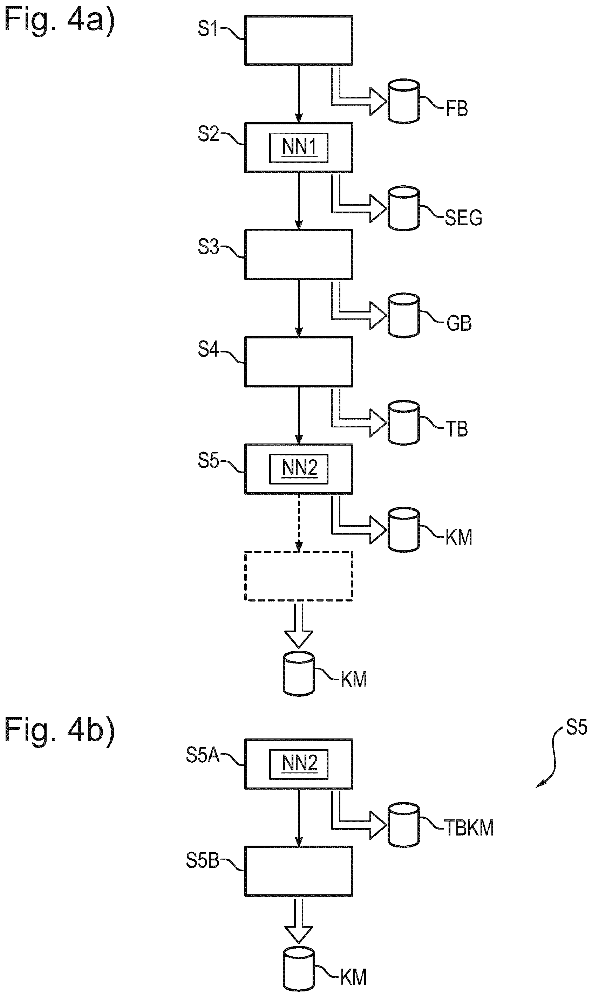

Die Figur 4a zeigt eine bevorzugte Abfolge von Schritten zur Durchführung eines hierin offenbarten Verfahrens. In einem Schritt S1 erfolgt ein Inkubieren des Zellsubstrates mit einer flüssigen Patientenprobe, welche potentiell primäre Antikörper aufweist, sowie ferner mit sekundären Antikörpern, welche mit einem Fluoreszenzfarbstoff markiert sind, ferner ein Bestrahlen des Zellsubstrates mit einer Anregungs-strahlung sowie ferner ein Erfassen des Immunfluoreszenzbildes.

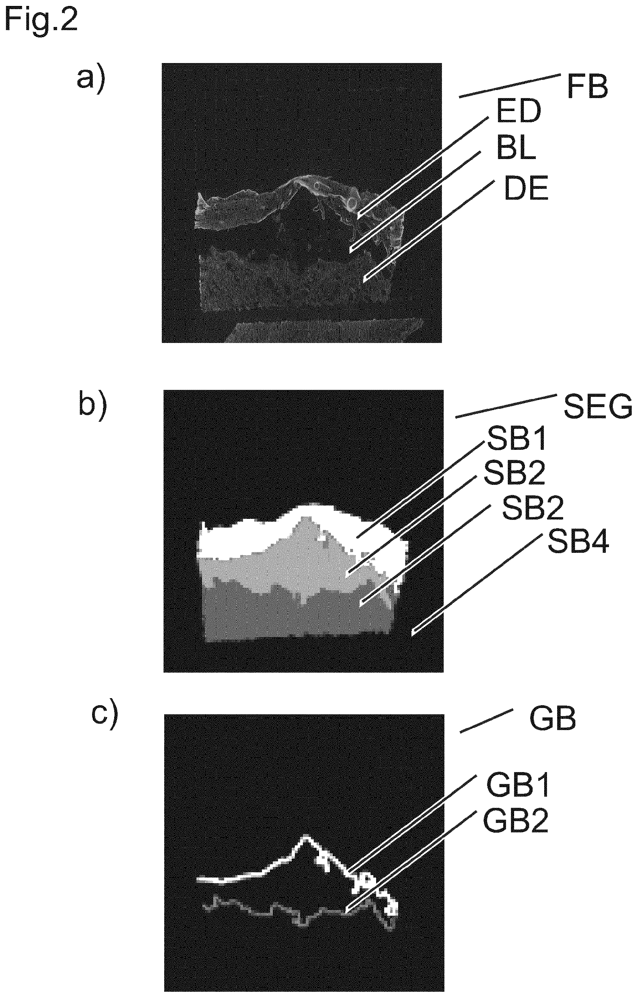

Die weiteren folgenden Schritte S2 bis S5 sind Schritte eines digitalen Bildverarbeitungsverfahrens, welche als Schritte SD zusammengefasst werden können. In einem Schritt S2 wird unter Verwendung eines neuronalen Netzes NN1 eine Segmentierungsinformation SEG bestimmt. Dieses erfolgt auf Basis des Fluoreszenzbildes FB. Ein beispielhaftes Fluoreszenzbild FB ist in der Figur 2a dargestellt. Klar zu erkennen ist der Bereich der Epidermis ED, der Bereich der Dermis DE sowie der Bereich des Zwischenraumes bzw. der Blase BL. In dem Schritt S2 wird nun die Segmentierungsinformation SEG bestimmt, welche beispielhaft in der Figur 2b dargestellt ist. Die Segmentierungsinformation SEG weist einen ersten Segmentierungsbereich SB1 auf, welcher den Zellsubstratbereich der Epidermis ED repräsentiert. Ferner weist die Segmentierungsinformation SEG einen zweiten Segmentierungsbereich SB2 auf, welcher vorzugsweise den Bereich BL des Zwischenraumes bzw. den Bereich der Blase repräsentiert. Vorzugsweise kann die Segmentierungsinformation SEG ferner einen dritten Segmentierungsbereich SB3 aufweisen, welcher einen dritten Zellsubstratbereich in Form der Dermis DE repräsentiert. Vorzugsweise weist die Segmentierungsinformation SEG ferner einen Segmentierungsbereich SB4 auf, welcher einen Hintergrund bzw. Background repräsentiert.

Dadurch, dass erfindungsgemäß die Segmentierungsinformation SEG durch ein erstes neuronales Netz bestimmt wird und dass dann das zweite neuronale Netz auf Basis mehrerer Teilbilder des Immunfluoreszenzbildes, welche mittels der Segmentierungsinformation SEG bzw. indirekt auf Basis der Segmentierungsinformation SEG bestimmt werden, das Konfidenzmaß der Präsenz des Fluoreszenzmusters bestimmt wird, muss das erste neuronale Netz lediglich darauf trainiert sein, Segmentierungsbereiche, welche jeweilige Zellsubstratbereiche repräsentieren, zu erkennen und als Information auszugeben. Später erst muss dann das zweite neuronale Netz auf Basis der selektierten Teilbilder das Konfidenzmaß der Präsenz des Fluoreszenzmusters bestimmen. Es muss also nicht ein einzelnes neuronales Netz die Erkennungsaufgabe des Erkennens der Substratbereiche bzw. Segmentierungsbereiche und auch die Detektionsaufgabe der Bestimmung des Konfidenzmaßes der Präsenz des Fluoreszenzmusters gleichzeitig leisten, sondern diese Teilaufgaben werden erfindungsgemäß auf zwei neuronale Netze in besonderer Weise verteilt. Daher ist das Verfahren besonders leistungsfähig.

Gemäß der Figur 4a erfolgt in einem Schritt S3 ein Bestimmen wenigstens eines Grenzbereiches, welcher einen Übergang von dem ersten Zellsubstratbereich hin zu dem zweiten Zellsubstratbereich in dem Fluoreszenzbild repräsentiert, auf Basis der Segmentierungsinformation SEG. Dieser bestimmte und detektierte Grenzbereich kann dann als eine Information GB durch den Schritt S3 bereitgestellt werden.

Die Figur 2c zeigt hierzu eine beispielhafte Grenzbereichsinformation GB, welche als einen Grenzbereich GB1 hier vorzugsweise ein Übergang von dem ersten Zellsubstratbereich in Form der Epidermis ED hin zu dem zweiten Zellsubstratbereich in Form des Zwischenraums bzw. der Blase BL indiziert.

Vorzugsweise kann in einem weiteren Schritt ein zweiter Grenzbereich GB2 bestimmt werden, welcher einen zweiten Übergang von den zweiten Zellsubstratbereich in Form des Zwischenraumes bzw. der Blase BL hin zu dem dritten Zellsubstratbereich in Form der Dermis DE repräsentiert.

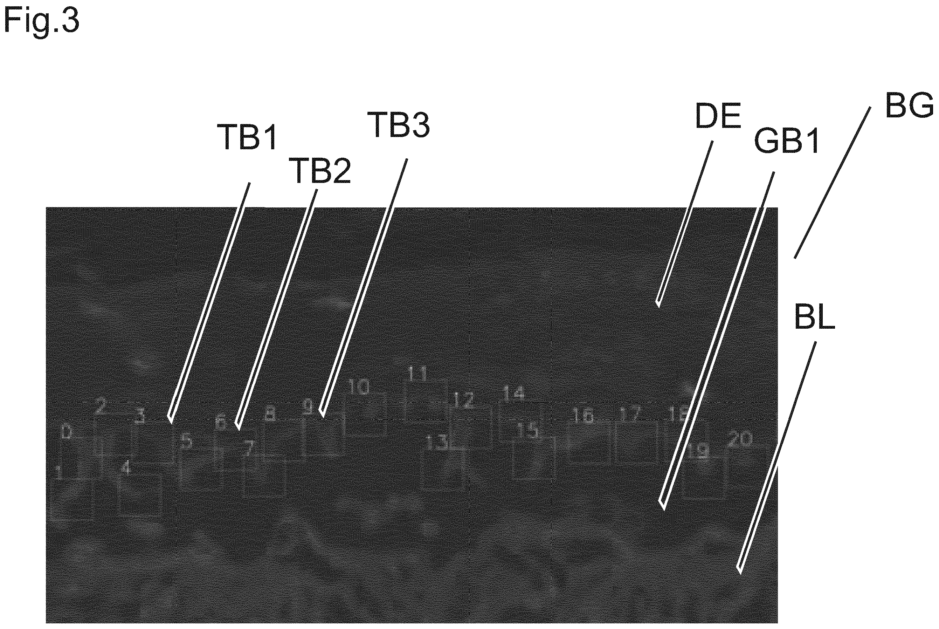

Gemäß der Figur 4a kann dann in einem Schritt S4 ein Selektieren mehrerer Teilbilder entlang des Grenzbereiches, insbesondere entlang des ersten Grenzbereiches GB1, erfolgen. Die Figur 3 zeigt hierzu beispielhaft für einen Ausschnitt FBA des Fluoreszenzbildes SB noch einmal den Bereich der Epidermis ED, den Bereich des Zwischenraumes bzw. der Blase BL als auch den Bereich GB1 als den Grenzbereich. Entlang des Grenzbereiches GB1 werden Teilbilder, wie beispielsweise die Teilbilder TB1, TB2, TB3, aus dem Fluoreszenzbild FB selektiert. Weitere Teilbilder sind hier mit Indizes von 0-20 in dem Bildausschnitt FBA indiziert.

Das Bestimmen des Konfidenzmaßes KM erfolgt gemäß der Figur 4a in dem Schritt S5 unter Verwendung des zweiten neuronalen Netzes NN2.

Dadurch, dass erfindungsgemäß nicht das gesamte Fluoreszenzbild FB durch das zweite neuronale Netz analysiert werden muss, sondern nur jeweilige Teilbilder, welche entlang des Grenzbereiches GB1 selektiert wurden, durch das zweite neuronale Netz analysiert werden, um das Konfidenzmaß zu bestimmen, muss das zweite neuronale Netz lediglich für Bildinformationen trainiert sein, welche durch Teilbilder eines Grenzbereiches, wie dem Grenzbereich GB1, gegeben sind. Daher kann das zweite neuronale Netz besonders sicher trainiert werden, um das Konfidenzmaß der Präsenz des Fluoreszenzmusters besonders sicher zu bestimmen. Würde ein vollständiges Fluoreszenzbild FB oder auch ein vollständiger Bildausschnitt FBA durch das zweite neuronale Netz vollständig analysiert werden, so würden deutlich mehr Informationen durch das zweite neuronale Netz verarbeitet werden müssen und somit höhere Freiheitsgrade im Training des zweiten neuronalen Netzes und auch im Entwurf des zweiten neuronalen Netzes zu berücksichtigen sein. Ein solches Vorgehen würde mit einer reduzierten Verlässlichkeit des Bestimmungsergebnisses des Konfidenzmaßes der Präsenz des Fluoreszenzmusters einhergehen.

In einem vorzugsweise durchzuführenden Schritt S6 erfolgt eine Ausgabe des Konfidenzmaßes KM. Das Konfidenzmaß kann eine Prozentangabe in einem Bereich von 0 bis 100% sein, welche das Konfidenzmaß präsentiert. Alternativ kann vorzugsweise das Konfidenzmaß ein Wert aus dem Wertebereich von 0 bis 1 sein. Gemäß einer weiteren bevorzugten Ausführungsform kann das Konfidenzmaß in Form einer Information "JA" oder "NEIN" ausgegeben werden.

Diese Ausgabe des Konfidenzmaßes KM kann als eine Ausgabe mittels einer Bereitstellung eines Datenelementes KM erfolgen. Alternativ kann diese Ausgabe erfolgen, indem auf einer Anzeigeeinheit einer Computereinheit das Konfidenzmaß dem Nutzer zur Anzeige gebracht wird.

Die Figur 4b zeigt eine bevorzugte Ausführungsform zur Durchführung des Schrittes S5, bei welchem in einem ersten Schritt S5A mittels des zweiten neuronalen Netzes NN2 zunächst für ein jeweiliges Teilbild ein jeweiliges Teilbildkonfidenzmaß bestimmt wird.

Anschließend wird in einem weiteren Schritt S5B das Konfidenzmaß KM auf Basis der Teilbildkonfidenzmaße TBKM bestimmt.

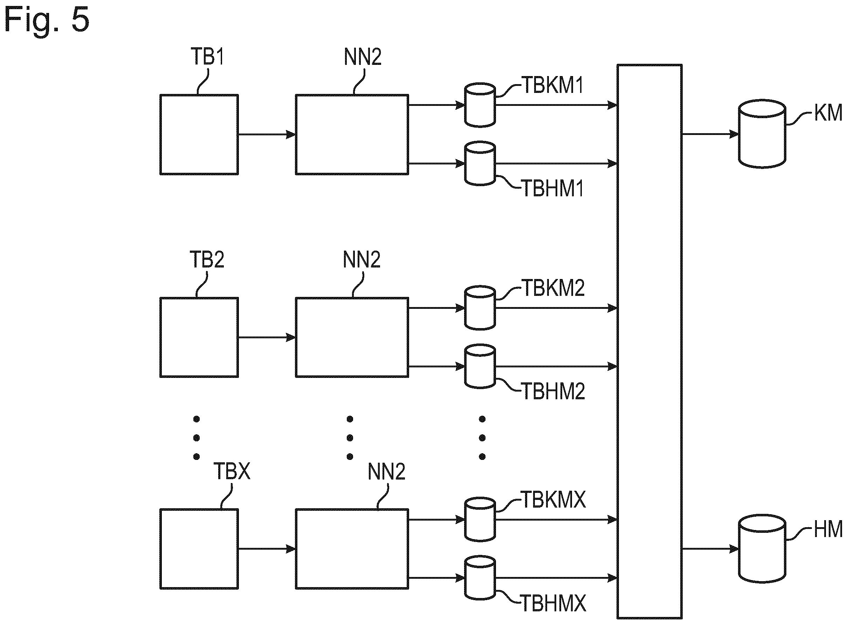

Die Figur 5 zeigt hierzu eine beispielhafte Struktur, bei welcher Teilbilder TB1, TB2, ..., TBX jeweils für sich bzw. jeweils individuell je Teilbild mittels des zweiten neuronalen Netzes NN2 separat analysiert werden. Ergebnis sind dann die jeweiligen Teilbildkonfidenzmaße TBKM1, TBKM2, ..., TBKMX. In einem weiteren Schritt S5B erfolgt dann die Bestimmung des Konfidenzmaßes KM auf Basis der Teilbildkonfidenzmaße TBKM.

Dadurch, dass das zweite neuronale Netz NN2 nur jeweils ein einzelnes Teilbild separat prozessiert, um ein jeweiliges Teilbildkonfidenzmaß zu bestimmen, kann das zweite neuronale Netz besonders spezifisch zur Analyse von Teilbildern TB1, TB2, ..., TX trainiert werden. Ferner muss das zweite neuronale Netz NN2 lediglich nur im Zuge der Analyse in einem Prozessierungsdurchlauf Bildinformationen eines einzelnen Teilbildes analysieren und eben nicht alle Bildinformationen aller Teilbilder in diesem Prozessierungsablauf gemeinsam betrachten. Daher ist das Verfahren besonders zuverlässig hinsichtlich der finalen Bestimmung des finalen Konfidenzmaßes KM.

Das Selektieren der Teilbilder TB1, TB2, ..., TBX entlang des Grenzbereiches GB1, wie in der Figur 3 dargestellt, kann vorzugsweise zufallsbasiert erfolgen. Insbesondere kann dieses Selektieren mittels Samplingverfahrens nach dem Poisson Disk-Verfahren erfolgen.

Einführung zur Bestimmung der Grenzbereiche:

Unter Betrachtung der Segmentierungsinformation SEG aus der Figur 2 kann zur Bestimmung der Grenzbereiche GB1, GB2 vorzugsweise auf folgendes Verfahren zurückgegriffen werden. Vorzugsweise wir ein jeweiliger Segmentierungsbereich SB1, ..., SB3 mittels einer Dilatation flächenmäßig erweitert. Es kann dann vorzugsweise zur Bestimmung des Grenzbereiches GB1, wie in Figur 2c dargestellt, eine Überlappung des mittels Dilatation erweiterten Bereiches SB1 mit wenigstens dem mittels Dilatation erweiterten Bereiches SB2 betrachtet werden. Vorzugsweise wird der Grenzbereich GB1 bestimmt, indem eine Überlappung des mittels Dilatation erweiterten Bereiches SB1 mit wenigstens einem der anderen mittels Dilatation erweiterten Bereiche SB2 oder SB3 betrachtet wird. Insbesondere kann eine Menge von Pixeln des Grenzbereiches GB1 anhand eines Dilatationsoperators und der jeweiligen Pixelmengen SB1, SB2, SB3 beschrie-ben werden als

$$GB1 = (DIL(SB1) \cap (DIL(SB2) \cup DIL(SB3))$$

Es kann dann vorzugsweise zur Bestimmung des Grenzbereiches GB2, wie in Figur 2c dargestellt, eine Überlappung des mittels Dilatation erweiterten Bereiches SB3 mit wenigstens dem mittels Dilatation erweiterten Bereiches SB2 betrachtet werden. Vorzugsweise wird der Grenzbereich GB2 bestimmt, indem eine Überlappung des mittels Dilatation erweiterten Bereiches SB3 mit wenigstens einem der anderen mittels Dilatation erweiterten Bereiche SB2 oder SB1 betrachtet wird. Insbesondere kann eine Menge von Pixeln des Grenzbereiches GB2 anhand eines Dilatationsoperators und der jeweiligen Pixelmengen SB1, SB2, SB3 beschrieben werden als

$$GB2 = (DIL(SB3) \cap (DIL(SB1) \cup DIL(SB2))$$

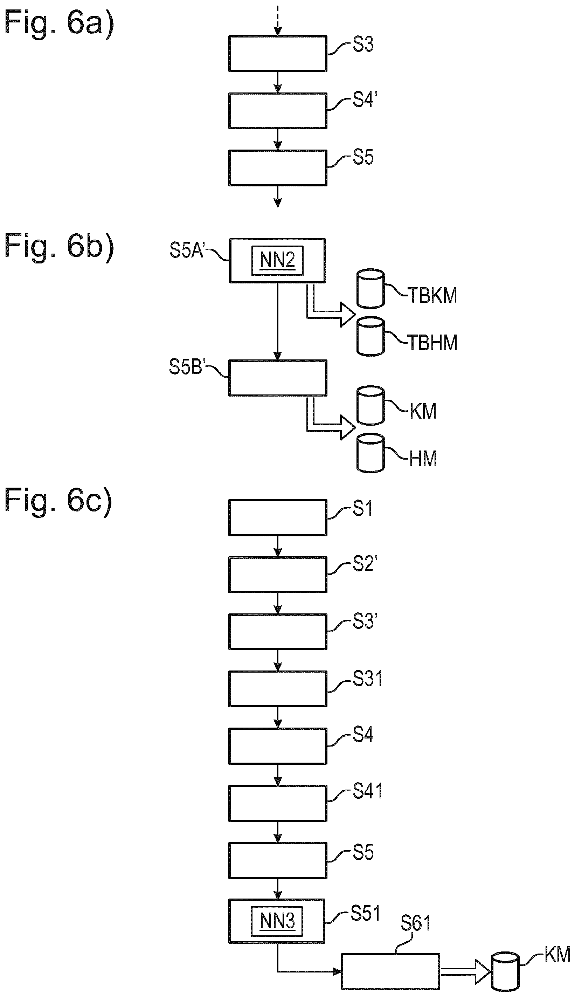

Das zufallsbasierte Selektieren der mehreren Teilbildbereiche wird in der Figur 6a durch einen modifizierten Schritt S4' repräsentiert.

Der Vorteil eines zufallsbasierten Selektierens der Teilbildbereich liegt daran, dass auch beim Training ein solches zufallsbasiertes Selektieren der Teilbildbereiche eine höhere Varianz der Daten der Teilbilder einbringt, um bei einer zu kleinen Trainingsmenge ein Overfitting zu vermeiden.

Gemäß der Figur 5 bestimmt das zweite neuronale Netz vorzugsweise für ein jeweiliges Teilbild ferner einen jeweiligen Helligkeitswert bzw. ein jeweiliges Helligkeitsmaß HM1, HM2, ... HMX. Dies kann, wie auch in der Figur 6b dargestellt, in einem modifizierten Schritt S5A' erfolgen.

Es kann dann gemäß der Figur 5 in einem modifizierten Schritt S5B', welcher auch in der Figur 6b dargestellt ist, auf Basis der jeweiligen Helligkeitswerte der Helligkeitswert bzw. der gesamte Helligkeitswert HM bestimmt werden.

Dieses ist vorteilhaft, da ein Bestimmen der Teilbildkonfidenzmaße und der Information der jeweiligen Helligkeitswerte in einem gemeinsamen neuronalen Netz NN2 eine bessere Generalisierung des Netzes erlaubt.

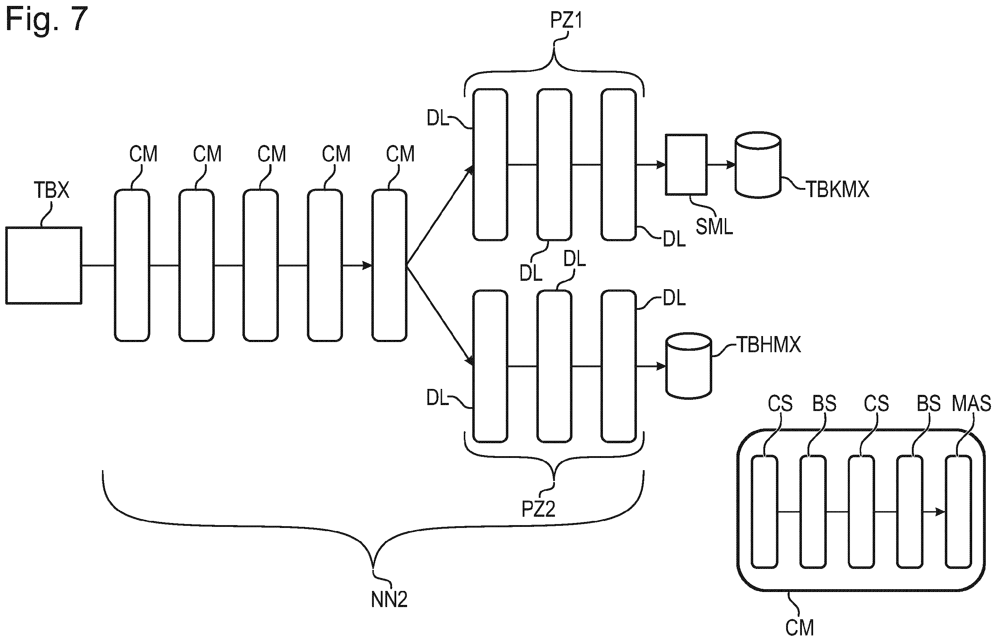

Die Figur 7 zeigt eine bevorzugte Struktur des neuronalen Netzes NN2. Das neuronale Netz NN2 analysiert ein jeweiliges Teilbild TB und bestimmt für ein jeweiliges Teilbild TB ein jeweiliges Teilbildkonfidenzmaß TBKMX. Ferner bestimmt das neuronale Netz NN2 vorzugsweise, wie bereits zuvor erläutert, ein jeweiliges Teilbildhelligkeitsmaß TBHM.

Hierfür kann das neuronale Netz NN2 auf eine Abfolge jeweiliger Convolutional Module CM zurückgreifen. Ein jeweiliges Convolution Modul CM weist hierbei vorzugsweise eine Abfolge eines Convolutional Steps CS, eines Batch Normalization Steps BS, eines weiteren Convolutional Steps CS sowie eines weiteren Batch Normalization Steps BS auf, gefolgt von einem Max Pooling Step MAS.

Mehrere solcher Convolutional Module CM können aufeinander folgen.

Auf das letzte Convolutional Modul kann dann in einem Prozessierungszweig PZ1 eine weitere Prozessierung mittels mehrerer Dense Layer DL , auch Fully Connected Layer genannt, erfolgen sowie schließlich mittels eines Softmax Layer SML das Teilbildkonfidenzmaß TBKMX zu bestimmen.

Vorzugsweise kann in einem weiteren Prozessierungszweig PZ2 eine Abfolge mehrerer Dense Layer DL2 erfolgen, um das Teilbildhelligkeitsmaß TBHM zu bestimmen.

Vorzugsweise wird für ein jedes Teilbild TBX mit Index 1=x...X ein solches Teilbildkonfidenzmaß TBKMX bestimmt, welches als ein Tupel $TBKM_x$ aus mehreren Labeln besteht gemäß

$$TBKM_x = \{ f_{pos}(TB_x), f_{neg}(TB_x), f_{uncl}(TB_x), f_{back}(TB_x) \}$$

mit

$f_{pos}(TB_x)$ Wahrscheinlichkeit, dass das Teilbild positiv ist (Fluoreszenz ist präsent)
$f_{neg}(TB_x)$ Wahrscheinlichkeit, dass das Teilbild negativ ist (Fluoreszenz nicht präsent)
$f_{uncl}(TB_x)$ Wahrscheinlichkeit, dass das Teilbild nicht zuordenbar ist
$f_{back}(TB_x)$ Wahrscheinlichkeit, dass das Teilbild einen Hintergrund zeigt ist

Weist ein Tupel $TBKM_x$ für ein Teilbild $TB_x$ als höchsten Wahrscheinlichkeitswert eine positive Wahrscheinlichkeit auf, so wird das Teilbild $TB_x$ als positiv eingestuft und der Menge P aller positiven Teilbilder zugeordnet. Weist ein Tupel $TBKM_x$ für ein Teilbild $TB_x$ als höchsten Wahrscheinlichkeitswert eine negative Wahrscheinlichkeit auf, so wird das Teilbild $TB_x$ als negativ eingestuft und der Menge N aller negativen Teilbilder zugeordnet. Weist ein Tupel $TBKM_x$ für ein Teilbild $TB_x$ als höchsten Wahrscheinlichkeitswert eine Wahrscheinlichkeit für die Klasse "nicht zuordenbar" auf, so wird das Teilbild $TB_x$ als nicht zuordenbar eingestuft. Weist ein Tupel $TBKM_x$ für ein Teilbild $TB_x$ als höchsten Wahrscheinlichkeitswert eine Wahrscheinlichkeit für die Klasse "Hintergrund" auf, so wird das Teilbild $TB_x$ als Hintergrund eingestuft. Teilbilder der Klassen "nicht zuordenbar" und "Hintergrund" werden im Weiteren nicht betrachtet.
Auf Basis der Teilbildkonfidenzmaße bzw. der Tupel $TBKM_x$ der Teilbilder $TB_x$, welche als positiv oder negativ eingestuft wurden, kann dann das Konfidenzmaß KM der Präsenz des Fluoreszenzmusters bestimmt werden als ein Skalarwert y im Wertebereich

$$y \in [0,1]$$

als

$$y = \frac{1 + y_{neg} - y_{pos}}{2}$$

mit

$$y_{neg} = \frac{\sum_{i \in N} f_{neg}(TB_i)}{|N|}$$

auf Basis aller negativen Teilbilder $TB_i$ aus der Menge N mit $i \in N$ und mit

$$y_{pos} = \frac{\sum_{i \in N} f_{pos}(TB_i)}{|P|}$$

auf Basis aller positiven Teilbilder $TB_i$ aus der Menge P mit $i \in P$.
Ferner werden zur Ermittlung des Helligkeitsmaßes HM als der Skalarwert b die drei Teilbilder $TB_i$ mit der höchsten Positivwahrscheinlichkeit $f_{pos}(TB_i)$ einer Menge P3 zugeordnet und betrachtet, indem deren Teilbildhelligkeitsmaße TBHM bzw. Teilhelligkeitswerte $b_i$ aggregiert werden gemäß

$$b = \frac{\sum_{i \in P} b_i}{|P_3|}$$

Die Figur 6c zeigt eine modifizierte Abfolge von Schritten des erfindungsgemäßen Verfahrens gemäß einer bevorzugten Ausführungsform. In dem dort vorhandenen zweiten Schritt S2' erfolgt das Bestimmen der Segmentierungsinformationen, welche den ersten, den zweiten und den dritten Segmentierungsbereich aufweist. Vorzugsweise sind die mehreren Teilbilder Teilbilder einer ersten Art.
Durch Verwendung des zuvor beschriebenen Schrittes S3 und eines weiteren Schrittes S31 erfolgt ein Bestimmen des ersten Grenzbereiches und eben auch ein Bestimmen des zweiten Grenzbereiches, welcher einen zweiten Übergang von dem zweiten Zellsubstratbereich hin zu dem dritten Zellsubstratbereich repräsentiert. Unter Durchführung des zuvor beschriebenen Schrittes S4 und Durchführung eines weiteren Schritt S41 erfolgt dann ferner eben auch ein Selektieren mehrerer Teilbilder zweiter Art aus dem Immunfloreszenzbild entlang des zweiten Grenzbereiches.

Unter Durchführung des zuvor beschriebenen Schrittes S5 als auch des weiteren, zusätzlichen Schrittes S51 erfolgt dann ein Bestimmen eines zweiten Konfidenzmaßes einer Präsenz eines zweiten Fluoreszenzmusters auf Basis der mehreren Teilbilder zweiter Art mittels eines dritten neuronalen Netzes NN3.

Das zweite Konfidenzmaß KM2 kann vorzugsweise in einem Schritt S61 ausgegeben werden.

Dadurch, dass in dieser bevorzugten Ausführungsform des Verfahrens sowohl ein Konfidenzmaß einer Präsenz eines Fluoreszenzmusters entlang des ersten Grenzbereiches als auch eines zweiten Konfidenzmaßes einer zweiten Präsenz eines zweiten Fluoreszenzmusters entlang des Grenzbereiches bestimmt wird und vorzugsweise auch ausgegeben werden kann, wird für bestimmte Fälle eine vorteilhafte Informationsgewinnung ermöglicht. Der erste Grenzbereich GB1 kann vorzugsweise als sogenanntes Blasendach bezeichnet werden und der zweite Grenzbereich vorzugsweise als sogenannter Blasenboden, wie auch in der Figur 1 eingehend erläutert.

Werden für beide Grenzbereiche Präsenzen von Fluoreszenzmustern bzw. deren Konfidenzmaße bestimmt und bereitgestellt bzw. ausgegeben, so kann vorzugsweise ein Kliniker diese Informationen in besonderer Weise auswerten. Ist ein Fluoreszenzmuster prinzipiell nur auf dem Blasendach präsent, so kann es sich dann wahrscheinlich um ein bullöses Pemphigoid handeln. Fluoresziert nur der Blasenboden, so kommen seltenere Pemphigoiderkrankungen außerhalb der Gruppe des bullösen Pemphigoids in Frage, also insbesondere eine nicht-bullöse Pemphigoiderkrankung, wie zum Beispiel die Epidermolysis bullosa acquisita.

Gibt es eine Fluoreszenz entlang des Blasenbodens und des Blasendachs, also entlang beider Grenzbereiche, so kommt prinzipiell eine Pemphigoiderkrankung in Frage.

Diese besondere Ausführungsform des vorgeschlagenen Verfahrens ist also hilfreich, um weiter hinsichtlich möglicher primärer Antikörper bzw. derer Präsenzen in der Patientenprobe differenzieren zu können, welches möglicherweise für eine Differenzierung in der Diagnostik für den Kliniker von Vorteil sein kann.

Das hierin vorgeschlagene Verfahren ist also insbesondere ein Verfahren zur digitalen Bildverarbeitung geeignet zur Detektion wenigstens eines Fluoreszenzmusters auf einem Immunfloreszenzbild eines biologischen Zellsubstrats zur Informationsgewinnung hinsichtlich einer möglichen Pemphigoiderkrankung. In einer besonderen Ausführungsform ist das Verfahren ein Verfahren zur Informationsgewinnung hinsichtlich einer möglichen bullösen Pemphigoiderkrankung. In einer weiteren besonderen Ausführungsform ist das Verfahren ein Verfahren zur Informationsgewinnung hinsichtlich einer möglichen nicht-bullösen Pemphigoiderkrankung. In noch einer weiteren besonderen Ausführungsform ist das Verfahren ein Verfahren zur Informationsgewinnung hinsichtlich einer möglichen nicht-bullösen Pemphigoiderkrankung oder bullösen Pemphigoiderkrankung.

Das biologische Zellsubstrat ist vorzugsweise ein Hautsubstrat eines Primaten. Das Hautsubstrat eines Primaten ist vorzugsweise ein Hautsubstrat eines Affen. Das Hautsubstrat eines Primaten eist vorzugsweise eine Epidermis und eine Dermis auf. Das Hautsubstrat eines Primaten ist vorzugsweise eine sogenannte Salt-Split Skin mit einer Blase zwischen Epidermis und Dermis.

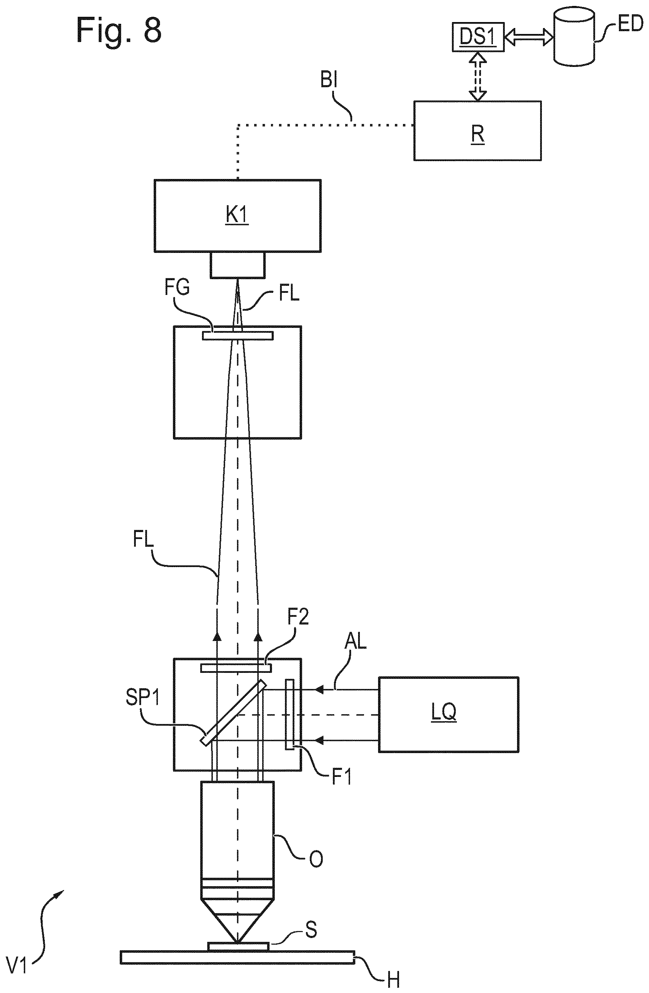

Figur 8 zeigt eine Vorrichtung V1, mittels welcher das offenbarte Verfahren vorzugsweise durchgeführt werden kann. Die Vorrichtung V1 kann als ein Fluoreszenzmikroskop bezeichnet werden. Die Vorrichtung V1 weist eine Halterung H für ein Substrat S bzw. einen Objektträger mit einem solche Substrat S auf, welches bzw. welcher in der zuvor beschriebenen Weise inkubiert wurde. Über eine Optik O wird Anregungslicht AL einer Anregungslichtquelle LQ hin zu dem Substrat S geleitet. Sich ergebende Fluoreszenzstrahlung FL wird dann durch die Optik O zurücktransmittiert und passiert den dichroitischen Spiegel SP1 und einen optionalen optischen Filter F2. Vorzugsweise passiert die Fluoreszenzstrahlung FL einen optischen Filter FG, welcher einen Grünkanal herausfiltert. Eine Kamera K1 ist vorzugsweise eine Monochromkamera, welche dann bei Vorhandensein eines optischen Filters FG die Fluoreszenzstrahlung FL in einem Grünkanal erfasst. Gemäß einer alternativen Ausführungsform ist die Kamera K1 eine Farbbildkamera, welche ohne Verwendung des optischen Filters FG auskommt und mittels einer Bayer-Matrix das Fluoreszenzbild in dem entsprechenden Farbkanal als einen Grünkanal erfasst. Die Kamera K1 stellt die Bildinformation BI bzw. das Fluoreszenzbild an eine Recheneinheit R bereit, welche diese Bildinformation BI verarbeitet. Vorzugsweise kann die Recheneinheit R über eine Datenschnittstelle DS1 Daten DE wie beispielsweise ein Fluoreszenzbild und/oder Konfidenzmaße ausgeben bzw. bereitstellen.

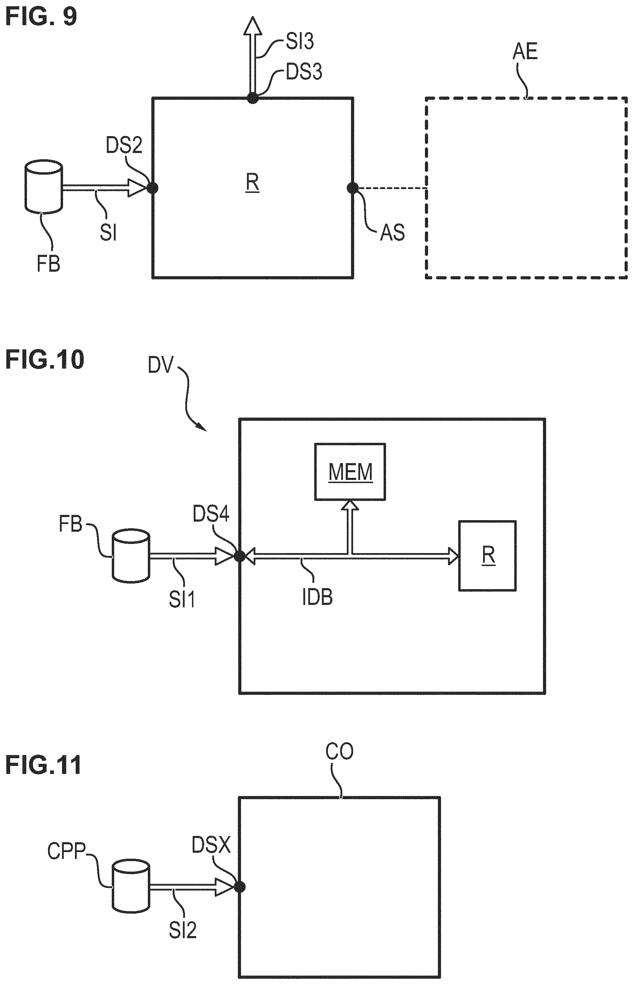

Figur 9 zeigt eine offenbarte Recheneinheit, welche vorzugsweise gemäß einer bevorzugten Ausführungsform ein Fluoreszenzbild FB als ein Datensignal SI über eine Datenschnittstelle DS2 entgegennimmt. Die Recheneinheit R kann dann die zuvor beschriebenen Informationen ermitteln und über eine Datenschnittstelle DS3 als ein Datensignal SI3 bereitstellen. Vorzugsweise kann dies über ein leitungsgebundenes oder drahtloses Datennetzwerk erfolgen. Besonders bevorzugt weist die Recheneinheit R eine Ausgabeschnittstelle AS auf zur Ausgabe der Informationen über eine Ausgabeeinheit AE. Die Ausgabeeinheit AE ist vorzugsweise eine Anzeigeeinheit für eine optische Anzeige der zuvor genannten Informationen.

Figur 10 zeigt eine erfindungsgemäße Datennetzwerkvorrichtung DV gemäß einer bevorzugten Ausführungsform. Die Datennetzwerkvorrichtung DV nimmt das Fluoreszenzbild FB als ein Datensignal SI1 über eine Datenschnittstelle DS4 entgegen. Die Datennetzwerkvorrichtung DV weist eine zuvor beschriebene Recheneinheit R auf sowie eine Speichereinheit MEM. Die Recheneinheit R, eine Speichereinheit MEM als auch die Datenschnittstelle DS4 werden vorzugsweise über einen internen Datenbus IDB miteinander verbunden.

Die Figur 11 zeigt eine Ausführungsform eines vorgeschlagenen Computerprogrammprodukts CPP. Das Computerprogrammprodukt CPP kann sein Datensignal SI2 über eine Datenschnittstelle DSX durch einen Computer CO entgegengenommen werden.

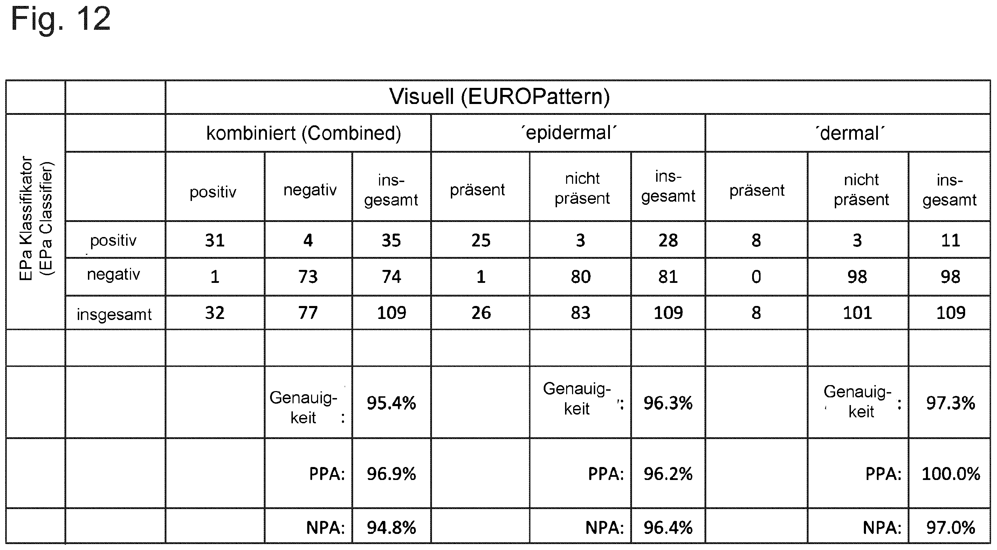

Figur 12 zeigt Experimentalergebnisse unter dem Label "Combined" für das Zellsubstrat des Salt-split Skin für den positiven Fall, dass in wenigstens einem der beiden Grenzbereiche GB1, insbesondere dem Blasendach, oder GB2, insbesondere Blasendboden, ein Fluoreszenzmuster präsent ist. Dies schließt den Fall mit ein, dass in beiden Grenzbereichen GB1, GB2 eine Fluoreszenz präsent ist. Ist in gar keinem Grenzbereich GB1, GB2 eine Fluoreszenz präsent, so wird der Fall als negativ bewertet. Von 32 tatsächlich positiven Fluoreszenzbildern wurden durch das erfindungsgemäße Verfahren "EPa-Classifier" 31 Bilder als positiv erkannt und 1 Bilder als negativ. Von 77 tatsächlich negativen Fluoreszenzbildern wurden durch das erfindungsgemäße Verfahren "EPa-Classifier" 4 Bilder als positiv erkannt und 73 Bilder als negativ. Es gab also insgesamt ("total") 109 Bilder, von welchen 35 als positiv und 74 als negativ erkannt wurden. Die Genauigkeit ("Accuracy") betrug 95,4%. Die Sensitivität ("PPA") betrug 96,9%. Die Spezifität ("NPA") betrug 94,8%.

Figur 12 zeigt ferner Experimentalergebnisse unter dem Label "epidermal" für das Zellsubstrat des Salt-split Skin für den positiven Fall, dass in dem ersten Grenzbereich GB1, insbesondere dem Blasendach, ein Fluoreszenzmuster präsent ist. Dies schließt den Fall mit ein, dass in beiden Grenzbereichen GB1, GB2 eine Fluoreszenz präsent ist. Ist in dem Grenzbereich GB1 kein Fluoreszenzmuster präsent, so wird der Fall als negativ bewertet. Von 26 tatsächlich positiven Fluoreszenzbildern wurden durch das erfindungsgemäße Verfahren "EPa-Classifier" 25 Bilder als positiv erkannt und 1 Bilder als negativ. Von 83 tatsächlich negativen Fluoreszenzbildern wurden durch das erfindungsgemäße Verfahren "EPa-Classifier" 3 Bilder als positiv erkannt und 80 Bilder als negativ. Es gab also insgesamt ("total") 109 Bilder, von welchen 28 als positiv und 81 als negativ erkannt wurden. Die Genauigkeit ("Accuracy") betrug 96,3%. Die Sensitivität ("PPA") betrug 96,2%. Die Spezifität ("NPA") betrug 96,4%.

Figur 12 zeigt ferner Experimentalergebnisse unter dem Label "dermal" für das Zellsubstrat des Salt-split Skin für den positiven Fall, dass in dem zweiten Grenzbereich GB2, insbesondere dem Blasenboden, ein Fluoreszenzmuster präsent ist. Dies schließt den Fall mit ein, dass in beiden Grenzbereichen GB1, GB2 eine Fluoreszenz präsent ist. Von 8 tatsächlich positiven Fluoreszenzbildern wurden durch das erfindungsgemäße Verfahren "EPa-Classifier" 8 Bilder als positiv erkannt und 0 Bilder als negativ. Von 101 tatsächlich negativen Fluoreszenzbildern wurden durch das erfindungsgemäße Verfahren "EPa-Classifier" 3 Bilder als positiv erkannt und 98 Bilder als negativ. Es gab also insgesamt ("total") 109 Bilder, von welchen 11 als positiv und 98 als negativ erkannt wurden. Die Genauigkeit ("Accuracy") betrug 97,3%. Die Sensitivität ("PPA") betrug 100,0%. Die Spezifität ("NPA") betrug 97,0%.

Je nach bestimmten Implementierungsanforderungen können Ausführungsbeispiele der Erfindung in Hardware oder in Software implementiert sein. Die Implementierung kann unter Verwendung eines digitalen Speichermediums, beispielsweise einer Floppy-Disk, einer DVD, einer Blu-Ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einer programmierbaren Hardwarekomponente derart zusammenwirken können oder zusammenwirken, dass das jeweilige Verfahren durchgeführt wird.

Eine programmierbare Hardwarekomponente wie insbesondere eine Recheneinheit kann durch einen Prozessor, einen Computerprozessor (CPU = Central Processing Unit), einen Grafikprozessor (GPU = Graphics Processing Unit), einen Computer, ein Computersystem, einen anwendungsspezifischen integrierten Schaltkreis (ASIC = Application-Specific Integrated Circuit), einen integrierten Schaltkreis (IC = Integrated Circuit), ein Ein-Chip-System (SOC = System on Chip), ein programmierbares Logikelement oder ein feldprogrammierbares Gatterarray mit einem Mikroprozessor (FPGA = Field Programmable Gate Array) gebildet sein.

Das digitale Speichermedium kann daher maschinen- oder computerlesbar sein. Manche Ausführungsbeispiele umfassen also einen Datenträger, der elektronisch lesbare Steuersignale aufweist, die in der Lage sind, mit einem programmierbaren Computersystem oder einer programmierbare Hardwarekomponente derart zusammenzuwirken, dass eines der hierin beschriebenen Verfahren durchgeführt wird. Ein Ausführungsbeispiel ist somit ein Datenträger (oder ein digitales Speichermedium oder ein computerlesbares Medium), auf dem das Programm zum Durchführen eines der hierin beschriebenen Verfahren aufgezeichnet ist.

Allgemein können Ausführungsbeispiele der vorliegenden Erfindung als Programm, Firmware, Computerprogramm oder Computerprogrammprodukt mit einem Programmcode oder als Daten implementiert sein, wobei der Programmcode oder die Daten dahin gehend wirksam ist bzw. sind, eines der Verfahren durchzuführen, wenn das Programm auf einem Prozessor oder einer programmierbaren Hardwarekomponente abläuft. Der Programmcode oder die Daten kann bzw. können beispielsweise auch auf einem maschinenlesbaren Träger oder Datenträger gespeichert sein. Der Programmcode oder die Daten können unter anderem als Quellcode, Maschinencode oder Bytecode sowie als anderer Zwischencode vorliegen.

Ein weiteres Ausführungsbeispiel ist ferner ein Datenstrom, eine Signalfolge oder eine Sequenz von Signalen, der bzw. die das Programm zum Durchführen eines der hierin beschriebenen Verfahren darstellt bzw. darstellen. Der Datenstrom, die Signalfolge oder die Sequenz von Signalen kann bzw. können beispielsweise dahin gehend konfiguriert sein, um über eine Datenkommunikationsverbindung, beispielsweise über das Internet oder ein anderes Netzwerk, transferiert zu werden. Ausführungsbeispiele sind so auch Daten repräsentierende Signalfolgen, die für eine Übersendung über ein Netzwerk oder eine Datenkommunikationsverbindung geeignet sind, wobei die Daten das Programm darstellen.

Ein Programm gemäß eines Ausführungsbeispiels kann eines der Verfahren während seiner Durchführung beispielsweise dadurch umsetzen, dass dieses Speicherstellen ausliest oder in diese ein Datum oder mehrere Daten hinein schreibt, wodurch gegebenenfalls Schaltvorgänge oder andere Vorgänge in Transistorstrukturen, in Verstärkerstrukturen oder in anderen elektrischen, optischen, magnetischen oder nach einem anderen Funktionsprinzip arbeitenden Bauteile hervorgerufen werden. Entsprechend können durch ein Auslesen einer Speicherstelle Daten, Werte, Sensorwerte oder andere Informationen von einem Programm erfasst, bestimmt oder gemessen werden. Ein Programm kann daher durch ein Auslesen von einer oder mehreren Speicherstellen Größen, Werte, Messgrößen und andere Informationen erfassen, bestimmen oder messen.

**Patentansprüche**

1. Verfahren zur digitalen Bildverarbeitung, aufweisend

    - Bereitstellen eines Immunfluoreszenzbilder (FB), welches eine Färbung eines biologischen Zellsubstrats durch einen Fluoreszenzfarbstoff repräsentiert,
    - Bestimmen einer Segmentierungsinformation (SEG) aufweisend wenigstens einen ersten und einen zweiten Segmentierungsbereich (SEG1, SEG2), wobei die Segmentierungsbereiche jeweils einen jeweiligen Zellsubstratbereich (D, BL) repräsentieren, mittels Segmentieren des Immunfluoreszenzbildes (FB) unter Verwendung eines ersten neuronalen Netzes (NN1),
    - Bestimmen eines Grenzbereiches (GB1), welcher einen Übergang von dem ersten Zellsubstratbereich (D) hin zu dem zweiten Zellsubstratbereich (BL) in dem Fluoreszenzbild (FB) repräsentiert, auf Basis der Segmentierungsinformation (SEG),
    - Selektieren mehrerer Teilbilder (TB1, ..., TBX) aus dem Immunfluoreszenzbild (FB) entlang des Grenzbereiches (GB1),
    - Bestimmen eines Konfidenzmaßes (KM) einer Präsenz des Fluoreszenzmusters auf Basis der mehreren Teilbilder (TB1, ..., TBX) mittels eines zweiten neuronalen Netzes (NN2).

2. Verfahren nach Anspruch 1,
    ferner aufweisend Ausgeben des Konfidenzmaßes (KM).

3. Verfahren nach Anspruch 1,
    ferner aufweisend

    - Bestimmen eines jeweiligen Teilbild-Konfidenzmaßes (TBKM1, ..., TBKMX) für ein jeweiliges Teilbild (TB1, ..., TBX) mittels des zweiten neuronalen Netzes (NN2)
    - sowie Bestimmen des Konfidenzmaßes (KM) auf Basis der Teilbild-Konfidenzmaße (TBKMX).

4. Verfahren nach Anspruch 1,
    ferner aufweisend zufallsbasiertes Selektieren der mehreren Teilbilder (TB1, ..., TBX) aus dem Immunfluoreszenzbild (FB) entlang des Grenzbereiches (GB1).

5. Verfahren nach Anspruch 1,
    ferner aufweisend

    - Bestimmen eines jeweiligen Helligkeitswertes (TBHM1, ..., TBHMX) für ein jeweiliges Teilbild (TB1, ..., TBX) mittels des zweiten neuronalen Netzes (NN2),
    - Bestimmen eines Gesamthelligkeitswertes (HM) auf Basis der Helligkeitswerte.

6. Verfahren nach Anspruch 1,

wobei die mehreren Teilbilder (TB1, ..., TBX) Teilbilder einer ersten Art sind,
ferner aufweisend

- Bestimmen der Segmentierungsinformation (SEG) aufweisend wenigstens den ersten, den zweiten und ferner einen dritten Segmentierungsbereich (SEG1, SEG2, SEG3), welcher einen dritten Zellsubstratbereich (DE) repräsentiert, mittels Segmentieren des Immunfluoreszenzbildes (FB) unter Verwendung des ersten neuronalen Netzes (NN1),
- Bestimmen eines zweiten Grenzbereiches (GB2), welcher einen zweiten Übergang von dem zweiten Zellsubstratbereich (BL) hin zu dem dritten Zellsubstratbereich (DE) in dem Fluoreszenzbild (FB) repräsentiert, auf Basis der Segmentierungsinformation (SEG),
- Selektieren mehrerer Teilbilder einer zweiten Art aus dem Immunfluoreszenzbild entlang des zweiten Grenzbereiches (GB2),
- Bestimmen eines zweiten Konfidenzmaßes einer Präsenz eines zweiten Fluoreszenzmusters auf Basis der mehreren Teilbildern der zweiten Art mittels eines dritten neuronalen Netzes (NN3).

7. Computerprogrammprodukt (CPP), umfassend Befehle, die bei der Ausführung des Programms durch einen Computer diesen veranlassen, das Verfahren zur digitalen Bildverarbeitung nach Anspruch 1 durchzuführen.

8. Datenträgersignal (SI2), welches das Computerprogrammprodukt (CPP) nach Anspruch 7 überträgt.

9. Vorrichtung zur Detektion wenigstens eines Fluoreszenzmusters auf einem Immunfluoreszenzbild (FB) eines biologischen Zellsubstrats,
aufweisend

- eine Haltevorrichtung (H) für einen Objektträger mit dem Zellsubstrat(S), welches mit einer Patientenprobe, aufweisend die Autoantikörper, sowie ferner mit sekundären Antikörpern, welche jeweils mit einem Fluoreszenzfarbstoff markiert sind, inkubiert wurde,
- wenigstens eine Bilderfassungseinheit (K1) zum Erfassen eines Fluoreszenzbildes (SG) des Zellsubstrats (S)

sowie ferner aufweisend wenigstens eine Recheneinheit (R), welche ausgebildet ist, die Schritte auszuführen

- Bestimmen einer Segmentierungsinformation (SEG) aufweisend wenigstens einen ersten und einen zweiten Segmentierungsbereich (SEG1, SEG2), wobei die Segmentierungsbereiche jeweils einen jeweiligen Zellsubstratbereich repräsentieren, mittels Segmentieren des Immunfluoreszenzbildes (FB) unter Verwendung eines ersten neuronalen Netzes (NN1),
- Bestimmen eines Grenzbereiches (GB1), welcher einen Übergang von dem ersten Zellsubstratbereich (D) hin zu dem zweiten Zellsubstratbereich (BL) in dem Fluoreszenzbild (FB) repräsentiert, auf Basis der Segmentierungsinformation (SEG),
- Selektieren mehrerer Teilbilder (TB1, ..., TBX) aus dem Immunfluoreszenzbild (FB) entlang des Grenzbereiches (GB1),
- Bestimmen eines Konfidenzmaßes (KM) einer Präsenz des Fluoreszenzmusters auf Basis der mehreren Teilbilder (TB1, ..., TBX) mittels eines zweiten neuronalen Netzes (NN2).

10. Datennetzwerkvorrichtung (DV),

aufweisend wenigstens eine Datenschnittstelle (DS4) zum Entgegennehmen eines Fluoreszenzbildes (FB), welches eine Färbung eines Zellsubstrats durch einen Fluoreszenzfarbstoff repräsentiert,
sowie ferner aufweisend wenigstens eine Recheneinheit (R), welche ausgebildet ist im Zuge einer digitalen Bildverarbeitung die Schritte auszuführen

- Bestimmen einer Segmentierungsinformation (SEG) aufweisend wenigstens einen ersten und einen zweiten Segmentierungsbereich (SEG1, SEG2), wobei die Segmentierungsbereiche jeweils einen jeweiligen Zellsubstratbereich repräsentieren, mittels Segmentieren des Immunfluoreszenzbildes (FB) unter Verwendung eines ersten neuronalen Netzes (NN1),
- Bestimmen eines Grenzbereiches (GB1), welcher einen Übergang von dem ersten Zellsubstratbereich (D) hin zu dem zweiten Zellsubstratbereich (BL) in dem Fluoreszenzbild (FB) repräsentiert, auf Basis der Segmentierungsinformation (SEG),
- Selektieren mehrerer Teilbilder (TB1, ..., TBX) aus dem Immunfluoreszenzbild (FB) entlang des Grenzbereiches (GB1),
- Bestimmen eines Konfidenzmaßes (KM) einer Präsenz des Fluoreszenzmusters auf Basis der mehreren Teilbilder (TB1, ..., TBX) mittels eines zweiten neuronalen Netzes (NN2).

**Claims**

1. Method for digital image processing, comprising

   - providing an immunofluorescence image (FB), which represents a staining of a biological cell substrate by a fluorescence stain,
   - determining segmentation information (SEG) comprising at least one first and one second segmentation area (SEG1, SEG2), wherein the segmentation areas each represent a respective cell substrate area (D, BL), via segmentation of the immunofluorescence image (FB) using a first neural network (NN1),
   - determining a boundary area (GB1), which represents a transition from the first cell substrate area (D) towards the second cell substrate area (BL) in the fluorescence image (FB), on the basis of the segmentation information (SEG),
   - selecting multiple partial images (TB1, ..., TBX) from the immunofluorescence image (FB) along the boundary area (GB1),
   - determining a confidence measure (KM) of a presence of the fluorescence pattern on the basis of the multiple partial images (TB1, ..., TBX) via a second neural network (NN2).

2. Method according to Claim 1,
   furthermore comprising outputting the confidence measure (KM).

3. Method according to Claim 1,
   furthermore comprising

   - determining a respective partial image confidence measure (TBKM1, ..., TBKMX) for a respective partial image (TB1, ..., TBX) via the second neural network (NN2)
   - and determining the confidence measure (KM) on the basis of the partial image confidence measures (TBKMX).

4. Method according to Claim 1,
   furthermore comprising randomly based selection of the multiple partial images (TB1, ..., TBX) from the immunofluorescence image (FB) along the boundary area (GB1).

5. Method according to Claim 1,
   furthermore comprising

   - determining a respective brightness value (TBHM1, ..., TBHMX) for a respective partial image (TB1, ..., TBX) via the second neural network (NN2),
   - determining an overall brightness value (HM) on the basis of the brightness values.

6. Method according to Claim 1,
   wherein the multiple partial images (TB1, ..., TBX) are partial images of a first type, furthermore comprising

   - determining the segmentation information (SEG), comprising at least the first, the second, and furthermore a third segmentation area (SEG1, SEG2, SEG3), which represents a third cell substrate area (DE), via segmentation of the immunofluorescence image (FB) using the first neural network (NN1),
   - determining a second boundary area (GB2), which represents a second transition from the second cell substrate area (BL) towards the third cell substrate area (DE) in the fluorescence image (FB), on the basis of the segmentation information (SEG),
   - selecting multiple partial images of a second type from the immunofluorescence image along the second boundary area (GB2),
   - determining a second confidence measure of a presence of a second fluorescence pattern on the basis of the multiple partial images of the second type via a third neural network (NN3).

7. Computer program product (CPP), comprising commands which, upon the execution of the program by a computer,

# EP 4 345 775 B1

prompt it to carry out the method for digital image processing according to Claim 1.

8. Data carrier signal (SI2), which transmits the computer program product (CPP) according to Claim 7.

9. Device for detecting at least one fluorescence pattern on an immunofluorescence image (FB) of a biological cell substrate,
comprising

- a holding device (H) for an object carrier having the cell substrate (S), which was incubated with a patient sample, including the autoantibodies, and furthermore with secondary antibodies, which are each marked using a fluorescence stain,
- at least one image capture unit (K1) for capturing a fluorescence image (SG) of the cell substrate (S)

and furthermore comprising at least one computing unit (R), which is designed to execute the following steps

- determining segmentation information (SEG) comprising at least one first and one second segmentation area (SEG1, SEG2), wherein the segmentation areas each represent a respective cell substrate area, via segmentation of the immunofluorescence image (FB) using a first neural network (NN1),
- determining a boundary area (GB1), which represents a transition from the first cell substrate area (D) towards the second cell substrate area (BL) in the fluorescence image (FB) on the basis of the segmentation information (SEG),
- selecting multiple partial images (TB1, ..., TBX) from the immunofluorescence image (FB) along the boundary area (GB1),
- determining a confidence measure (KM) of a presence of the fluorescence pattern on the basis of the multiple partial images (TB1, ..., TBX) via a second neural network (NN2).

10. Data network device (DV),

comprising at least one data interface (DS4) for accepting a fluorescence image (FB), which represents a staining of a cell substrate by a fluorescence stain,
and furthermore comprising at least one computing unit (R), which is designed to execute the following steps in the course of digital image processing

- determining segmentation information (SEG) comprising at least one first and one second segmentation area (SEG1, SEG2), wherein the segmentation areas each represent a respective cell substrate area, via segmentation of the immunofluorescence image (FB) using a first neural network (NN1),
- determining a boundary area (GB1), which represents a transition from the first cell substrate area (D) towards the second cell substrate area (BL) in the fluorescence image (FB), on the basis of the segmentation information (SEG),
- selecting multiple partial images (TB1, ..., TBX) from the immunofluorescence image (FB) along the boundary area (GB1),
- determining a confidence measure (KM) of a presence of the fluorescence pattern on the basis of the multiple partial images (TB1, ..., TBX) via a second neural network (NN2).

**Revendications**

1. Procédé de traitement d'image numérique, comprenant

- la fourniture d'une image immunofluorescente (FB) qui représente une coloration d'un substrat de cellule biologique par un colorant fluorescent,
- la détermination d'une information de segmentation (SEG) comprenant au moins une première et une deuxième zone de segmentation (SEG1, SEG2), les zones de segmentation représentant chacune une zone respective de substrat de cellule (D, BL), au moyen de la segmentation de l'image immunofluorescente (FB) à l'aide d'un premier réseau neuronal (NN1),
- la détermination d'une zone limite (GB1) qui représente une transition de la première zone de substrat cellulaire (D) vers la deuxième zone de substrat cellulaire (BL) dans l'image fluorescente (FB), sur la base de l'information de segmentation (SEG),

- la sélection de multiples sous-images (TB1, ..., TBX) à partir de l'image immunofluorescente (FB) le long de la zone limite (GB1),
- la détermination d'une mesure de confiance (KM) d'une présence du motif fluorescent sur la base des multiples sous-images (TB1, ..., TBX) au moyen d'un deuxième réseau neuronal (NN2).

2. Procédé selon la revendication 1,
comprenant en outre la fourniture en sortie de la mesure de confiance (KM).

3. Procédé selon la revendication 1,
comprenant en outre

- la détermination d'une mesure de confiance de sous-image respective (TBKM1, ..., TBKMX) pour une sous-image respective (TB1, ..., TBX) au moyen du deuxième réseau neuronal (NN2)
- ainsi que la détermination de la mesure de confiance (KM) sur la base des mesures de confiance de sous-image (TBKMX).

4. Procédé selon la revendication 1,
comprenant en outre la sélection aléatoire des multiples sous-images (TB1, ..., TBX) à partir de l'image immunofluorescente (FB) le long de la zone limite (GB1).

5. Procédé selon la revendication 1,
comprenant en outre

- la détermination d'une valeur de luminosité respective (TBHM1, ..., TBHMX) pour une sous-image respective (TB1, ..., TBX) au moyen du deuxième réseau neuronal (NN2),
- la détermination d'une valeur de luminosité globale (HM) sur la base des valeurs de luminosité.

6. Procédé selon la revendication 1,

dans lequel les multiples sous-images (TB1, ..., TBX) sont des sous-images d'un premier type, comprenant en outre

- la détermination de l'information de segmentation (SEG) comprenant au moins la première, la deuxième et en outre une troisième zone de segmentation (SEG1, SEG2, SEG3), qui représente une troisième zone de substrat de cellule (DE), au moyen d'une segmentation de l'image immunofluorescente (FB) à l'aide du premier réseau neuronal (NN1),
- la détermination d'une deuxième zone limite (GB2) qui représente une deuxième transition de la deuxième zone de substrat de cellule (BL) vers la troisième zone de substrat de cellule (DE) dans l'image fluorescente (FB), sur la base de l'information de segmentation (SEG),
- la sélection de multiples sous-images d'un deuxième type à partir de l'image immunofluorescente le long de la deuxième zone limite (GB2),
- la détermination d'une deuxième mesure de confiance d'une présence d'un deuxième motif fluorescent sur la base des multiples sous-images du deuxième type au moyen d'un troisième réseau neuronal (NN3).

7. Produit de programme informatique (CPP), comprenant des instructions qui, lors de l'exécution du programme par un ordinateur, l'amènent à mettre en œuvre le procédé de traitement d'image numérique selon la revendication 1.

8. Signal porteur de données (SI2), qui transmet le produit de programme informatique (CPP) selon la revendication 7.

9. Dispositif de détection d'au moins un motif fluorescent sur une image immunofluorescente (FB) d'un substrat de cellule biologique,
comprenant

- un dispositif de maintien (H) pour une lame porte-objet dotée du substrat de cellule (S), qui a été amené à incuber avec un échantillon de patient comprenant les auto-anticorps, et comprenant en outre des anticorps secondaires, qui sont respectivement marqués par un colorant fluorescent,
- au moins une unité d'acquisition d'image (K1) pour l'acquisition d'une image fluorescente (SG) du substrat de cellule (S)

et comprenant en outre au moins une unité de calcul (R), qui est conçue pour exécuter les étapes comprenant

- la détermination d'une information de segmentation (SEG) comprenant au moins une première et une deuxième zone de segmentation (SEG1, SEG2), les zones de segmentation représentant chacune une zone respective de substrat de cellule, au moyen d'une segmentation de l'image immunofluorescente (FB) à l'aide d'un premier réseau neuronal (NN1),
- la détermination d'une zone limite (GB1) qui représente une transition de la première zone de substrat de cellule (D) vers la deuxième zone de substrat de cellule (BL) dans l'image fluorescente (FB), sur la base de l'information de segmentation (SEG),
- la sélection de multiples sous-images (TB1, ..., TBX) à partir de l'image immunofluorescente (FB) le long de la zone limite (GB1),
- la détermination d'une mesure de confiance (KM) d'une présence du motif fluorescent sur la base des multiples sous-images (TB1, ..., TBX) au moyen d'un deuxième réseau neuronal (NN2).

10. Dispositif de réseau de données (DV),

comprenant au moins une interface de données (DS4) pour la réception d'une image fluorescente (FB), qui représente une coloration d'un substrat de cellule par un colorant fluorescent,
et comprenant en outre au moins une unité de calcul (R), qui est conçue pour exécuter, dans le cadre d'un traitement d'image numérique, les étapes comprenant

- la détermination d'une information de segmentation (SEG) comprenant au moins une première et une deuxième zone de segmentation (SEG1, SEG2), les zones de segmentation représentant chacune une zone respective de substrat de cellule, au moyen d'une segmentation de l'image immunofluorescente (FB) à l'aide d'un premier réseau neuronal (NN1),
- la détermination d'une zone limite (GB1) qui représente une transition de la première zone de substrat de cellule (D) vers la deuxième zone de substrat de cellule (BL) dans l'image fluorescente (FB), sur la base de l'information de segmentation (SEG),
- la sélection de multiples sous-images (TB1, ..., TBX) à partir de l'image immunofluorescente (FB) le long de la zone limite (GB1),
- la détermination d'une mesure de confiance (KM) d'une présence du motif fluorescent sur la base des multiples sous-images (TB1, ..., TBX) au moyen d'un deuxième réseau neuronal (NN2).

Fig.1

# Fig.2

Fig.3

EP 4 345 775 B1

Fig. 4a)

S1

FB

S2    NN1

SEG

S3

GB

S4

TB

S5    NN2

KM

KM

Fig. 4b)

S5

S5A    NN2

TBKM

S5B

KM

Fig. 5

EP 4 345 775 B1

Fig. 6a)

Fig. 6b)

Fig. 6c)

Fig. 7

# Fig. 8

**FIG. 9**

**FIG.10**

**FIG.11**

# Fig. 12

| EPa Klassifikator (EPa Classifier) | | Visuell (EUROPattern) | | | | | | | | |
|---|---|---|---|---|---|---|---|---|---|---|
| | | kombiniert (Combined) | | | ´epidermal´ | | | ´dermal´ | | |
| | | positiv | negativ | ins-gesamt | präsent | nicht präsent | ins-gesamt | präsent | nicht präsent | ins-gesamt |
| | positiv | 31 | 4 | 35 | 25 | 3 | 28 | 8 | 3 | 11 |
| | negativ | 1 | 73 | 74 | 1 | 80 | 81 | 0 | 98 | 98 |
| | insgesamt | 32 | 77 | 109 | 26 | 83 | 109 | 8 | 101 | 109 |
| | | | | | | | | | | |
| | | | Genauig-keit : | 95.4% | | Genauig-keit ´: | 96.3% | | Genauig-keit : | 97.3% |
| | | | PPA: | 96.9% | | PPA: | 96.2% | | PPA: | 100.0% |
| | | | NPA: | 94.8% | | NPA: | 96.4% | | NPA: | 97.0% |

EP 4 345 775 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20190371425 A1

- EP 4016082 A1

**In der Beschreibung aufgeführte Nicht-Patentliteratur**

- Automatic HEp-2 cell segmentation in indirect immunofluorescence images using deep learning. **JIANG GUAN-TING et al.** Spie smart structures and materials + nondestructive evaluation and health monitoring. Spie, 20 April 2021, 1179205-1179205